# EUROPEAN PATENT APPLICATION

(11) **EP 0 690 073 A1**
(43) Date of publication of application: **03.01.1996**
(21) Application number: 94304659.9
(22) Date of filing: 27.06.1994
(51) Int. Cl.: C08C 19/38

(54) **Saturated, aminated, alkoxylated polybutadienes**

(71) Applicant: HUNTSMAN CORPORATION, Austin, Texas 78761 (US)
(72) Inventor: Knifton, John Frederick, Austin, Texas 78726 (US); Cuscurida, Michael, Austin, Texas 78731 (US)
(74) Representative: Watkins, David

(57) **Abstract**

Saturated, aminated alkoxylated polymers having a high degree of maintain and saturation can be obtained by reductive amination of an alkyoxylated liquid polymer obtained by alkoxylating a hydroxyl-terminated polybutadiene (HTPB) with one or more alkylene oxides having at least three carbon atoms to provide secondary terminal hydroxyl groups, by contacting the alkoxylated liquid polymer is contacted with hydrogen and ammonia in the presence of a catalyst whose active components are nickel oxide, copper oxide, chromium oxide and molybdenum oxide, the molar ratio of hydrogen to ammonia to secondary hydroxyl groups on the alkoxylated liquid polymer being at least 50 : at least 10:1.

Preferred products include saturated, aminated alkoxylated polymers of the formula: 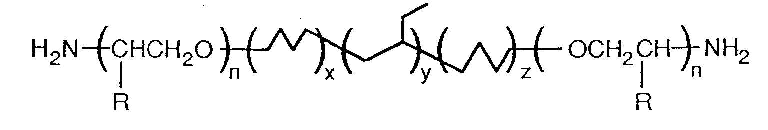
wherein each R is independently alkyl of 1 to 4 carbon atoms;
each n is independently from 1 to 100; and
x, y and z are numbers,
the number average molecular weight of the hydrocarbon backbone being from 200 to 5000.

## Description

This invention relates to polyoxyalkylene polyamines and, in one aspect, more particularly relates to saturated polyoxyalkylene polyamines made by simultaneously aminating and hydrogenating an alkoxylated, hydroxy-terminated polyalkene.

Low molecular weight homopolymers and copolymers of 1,3-dienes have been known for a long time. It is advantageous for many uses to alter the properties of the hydrophobic polymers in a controlled fashion, by the introduction of reactive groups, such as hydroxyl and amine groups. Typically, compounds terminated with these reactive groups may be used as curing agents for epoxy resins, as cross-linking agents or binders for textiles, and as intermediates in the preparation of polyurethanes and polyureas, including flexible urethane foams and urethane elastomers. Likewise, polyoxyalkylene polyamines falling within the above description are well known.

For example, US-A-4658062 relates to novel amine terminated polybutadiene compounds of the formula: 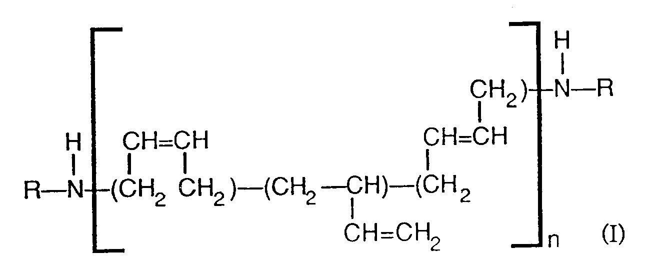
wherein R is hydrogen, a straight or branched chain alkyl group containing from 1 to 10 carbon atoms or a substituted or unsubstituted aryl or aralkyl group containing one or more benzenoid rings which may be fused or joined by single bonds, and n is 5 to 1500, and to a process for the preparation thereof. These materials are made by reacting an alkane- or arenesulphonate-terminated polybutadiene with a primary amine or ammonia. Further the process involves reacting a polyhydroxybutadiene homopolymer with an alkane- or arene-sulphonyl chloride or fluoride in the presence of a tertiary amine catalyst at a preferred temperature between 25 and 110°C. The process comprises a two-step procedure, involving an intermediate alkane- or arene-sulphonated polybutadiene.

US-A-4721754 describes a polybutadiene composition useful for the preparation of polyurea and/or polyurethane elastomers comprising a blend of polyhydroxybutadiene homopolymer and an amine-terminated polybutadiene. Similarly, US-A-4812524 describes novel polyurea/polyurethane two-component adhesive compositions which comprise the reaction product of (a) a blend of an amine terminated polybutadiene and a polyhydroxybutadiene with (b) an aliphatic or aromatic di-or polyisocyanate and optionally chain extenders, tackifiers, coupling agents, fillers and catalysts.

US-A-4994621 teaches that aminated, alkoxylated hydroxyl-terminated polymers can be made by a process involving first polymerizing one or more unsaturated hydrocarbons, such as butadiene, to form a liquid polymer. Next, the liquid polymer is provided with terminal hydroxyl groups. Then, the hydroxylated liquid polymer is alkoxylated with one or more alkylene oxides, such as ethylene oxide, propylene oxide, butylene oxide and mixtures thereof, to provide secondary terminal hydroxyl groups. Finally, the alkoxylated liquid polymer is aminated over a transition metal oxide catalyst, such as a combination of nickel oxide/copper oxide/chromium oxide. If the alkoxylated, hydroxyl-terminated liquid polymer is terminated with primary hydroxyl groups, then the amination does not proceed well. Although it is stated that "Typically, part or all of the double bonds are reduced in this amination procedure", the only structures of the aminated, alkoxylated hydroxyl-terminated polymers presented show the polybutadiene core to be unsaturated. Additionally, it was subsequently found that the end products of the process of US-A-4994621 retained a relatively high degree of unsaturation. This is confirmed by Examples 19 to 22 set out below.

Further unpublished U.S. Patent Application Serial No. 08/010528, describes the synthesis of novel amine derivatives of hydroxyl-terminated polybutadienes having the formula: 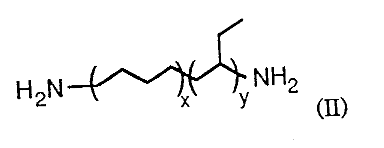
where x and y are numbers, formed by amination and hydrogenation of a hydroxyl-terminated polybutadiene oligomer, having an average molecular weight of 100 to 200,000 and a hydroxyl content of 0.1 to 20 meq/g, in the presence of an inert solvent over a catalyst comprising, Ni-Cu-Cr supported on alumina at a partial pressure of hydrogen sufficient to provide a flow rate of 0.062 to 62.06 m³/Kg (1 to 1,000 scf/lb) of hydroxyl-terminated polybutadiene (HTPB).

The term hydroxyl-terminated polybutadienes (HTPB) is used herein to cover hydroxyl-terminated copolymers where at least one of the monomers used is butadiene. This definition includes hydroxyl-terminated homopolymers of butadiene.

It is always desirable to produce polyamines with improved properties for specific uses. It would be desirable to produce aliphatic amine derivatives of alkoxylated polybutadienes terminated with secondary hydroxyl groups which possessed a high concentration of secondary alkyl primary amines, along with a high degree of saturation and a low equivalent weight of primary alkyl primary amines or secondary amines. Such compositions would be expected to be useful in RIM and polyurethane applications.

Accordingly, it is an object of the present invention to provide novel, saturated, aminated, alkoxylated polybutadienes.

It is another object of the present invention to provide a process for producing saturated, aminated, alkoxylated polybutadienes with a high degree of saturation and a high degree of amination.

The present invention provides a process for making saturated, aminated alkoxylated polymers by reductive amination of an alkoxylated liquid polymer obtained by alkoxylating a hydroxyl-terminated polybutadiene (HTPB) with one or more alkylene oxides having at least three carbon atoms to provide secondary terminal hydroxyl groups. According to the invention the alkoxylated liquid polymer is contacted with hydrogen and ammonia in the presence of a catalyst whose active components are nickel oxide, copper oxide, chromium oxide and molybdenum oxide, the molar ratio of hydrogen to ammonia to secondary hydroxyl groups on the alkoxylated liquid polymer being at least 50 : at least 10:1.

A preferred catalyst comprises 10 to 50 wt.% of nickel oxide, 1 to 20 wt.% of copper oxide, 0.1 to 10 wt.% of chromium oxide and 0.1 to 10 wt.% of molybdenum oxide. The catalyst is advantageously supported on alumina, silica, zirconia, magnesia, titania or a mixture thereof.

Preferably, the reductive amination is conducted in the presence of *t*-butanol as an inert solvent.

According to one embodiment, the resulting primary amine terminated, saturated derivatives of alkoxylated polybutadienes have the general structure: 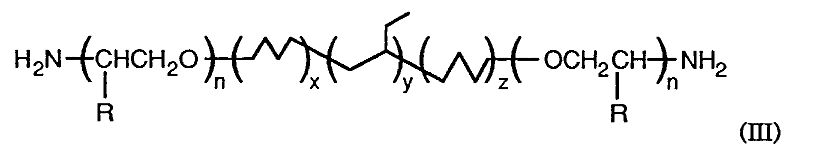
wherein each R is independently alkyl of 1 to 4 carbon atoms;
each n is independently from 1 to 100; and x y and z are numbers,
the number average molecular weight of the hydrocarbon backbone being from 200 to 5000.

The reaction sequence may be briefly summarized, in a non-limiting example, by noting that a hydroxy terminated polybutadiene of the formula set out below: 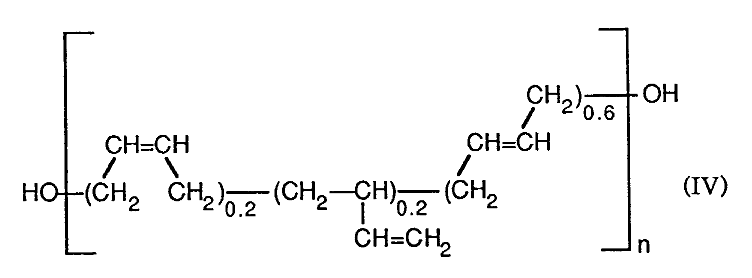
where n is 10 to 60, and the values 0.2 and 0.6 represent statistical distributions of isomer moieties by which butadiene can polymerize, is first alkoxylated and then simultaneously hydrogenated and aminated. The reaction intermediate is capped with an alkylene oxide having at least three carbon atoms, such as propylene oxide, or butylene oxide (all forms), otherwise the amination does not proceed well. In other words, the hydroxy-terminated polybutadiene that is alkoxylated should not be primary hydroxyl terminated.

The polyamines are hydrophobic and are useful as curing agents for epoxy resins, and in reaction injection moulding (RIM) elastomers. They may also be used in the preparation of flexible polyurea foams. Essentially all of the double bonds in the original polyol are reduced during the amination step. In addition, a higher degree of amination is achieved using the method of this invention. It is demonstrated in the Examples below that use of a Ni-Cu-Cr-Mb catalyst provides both more amination, and more reduction, and less oligomerization than a Ni-Cu-Cr catalyst according to US-A-4994621. The process for making the novel materials will be described in more detail below.

The monomer initiator for these primary amine hydrocarbon products should be a homopolymer or copolymer of butadiene. Suitable comonomers include isoprene; 1,4-pentadiene; 1,6-hexadiene; 1,7-octadiene; styrene; acrylonitrile; methacrylonitrile; α-methylstyrene; methylstyrene; 2,4-dimethylstyrene; ethylstyrene; isopropylstyrene; butylstyrene; substituted styrenes, such as cyanostyrene; phenylstyrene; cyclohexylstyrene; benzylstyrene; nitrostyrene; N,N-dimethylaminostyrene; acetoxystyrene; methyl 4-vinylbenzoate; phenoxystyrene; p-vinyl diphenyl sulphide; p-vinylphenyl phenyl oxide; acrylic and substituted acrylic monomers such as acrylic acid; methacrylic acid; methyl acrylate; 2-hydroxyethyl acrylate; 2-hydroxyethyl methacrylate; methyl methacrylate; cyclohexyl methacrylate; benzyl methacrylate; isopropyl methacrylate; octyl methacrylate; ethyl α-ethoxyacrylate; methyl α-acetoaminoacrylate; butyl acrylate; 2-ethylhexyl acrylate; phenyl acrylate; phenyl methacrylate; N,N-dimethylacrylamide; N,N-dibenzylacrylamide; N-butylacrylamide; methacryloyl formamide; vinyl esters; vinyl ethers; vinyl ketones; vinyl acetate; vinyl alcohol; vinyl butyrate; isopropenyl acetate; vinyl formate; vinyl acrylate; vinyl methacrylate; vinyl methoxy acetate; vinyl benzoate; vinyl toluene; vinyl naphthalene, vinyl methyl ether, vinyl ethyl ether; vinyl propyl ethers; vinyl butyl ethers; vinyl 2-ethylhexyl ether; vinyl phenyl ether; vinyl 2-methoxyethyl ether; methoxybutadiene; vinyl 2-butoxyethyl ether; 3,4-dihydro-1,2-pyran; 2-butoxy-2'-vinyloxy diethyl ether; vinyl 2-ethylmercaptoethyl ether; vinyl methyl ketone; vinyl ethyl ketone; vinyl phenyl ketone; vinyl ethyl sulphide; vinyl ethyl sulphone; N-methyl-N-vinyl acetamide; N-vinylpyrrolidone; vinyl imidazole; divinyl sulphide; divinyl sulphoxide; divinyl sulphone; sodium vinyl imidazole; sodium vinyl sulphonate; methyl vinyl sulphonate; N-vinyl pyrrole; dimethyl fumarate; dimethyl maleate; maleic acid; crotonic acid; fumaric acid; itaconic acid; monomethyl itaconate; *t*-butylaminoethyl methacrylate; dimethylaminoethyl methacrylate; glycidyl acrylate; allyl alcohol; glycol monoesters of itaconic acid; vinyl pyridine; maleic anhydride; maleimide; and N-substituted maleimides, such as N-phenylmaleimide.

The polymerization initiator catalyst may be any suitable initiator for the particular monomers employed. Suitable catalytic initiators are the free radial type of vinyl polymerization catalysts, such as the peroxides; persulphates; perborates; percarbonates; and azo compounds. Specific examples include hydrogen peroxide; 2,2'-azo-*bis*-isobutyronitrile (AIBN); dibenzoyl peroxide; lauroyl peroxide; di-*t*-butyl peroxide; diisopropyl peroxide carbonate; *t*-butyl peroxy-2-ethylhexanoate; *t*-butyl perneodecanoate; *t*-butyl perbenzoate; *t*-butyl percrotonate; *t*-butyl perisobutyrate; di-*t*-butyl perphthalate; 2,2'-azo-*bis*-(2-methylbutane-nitrile) for example. Other suitable catalysts may be employed, of course.

The polymerization of the butadiene may be carried out according to conventional, known procedures. The polymers are provided with hydroxyl groups at the terminal ends thereof also by any known technique.

The preferred polyamine precursor oligomer employed according to the instant invention is a hydroxyl-terminated polybutadiene oligomer (HTPB) represented by the structure: 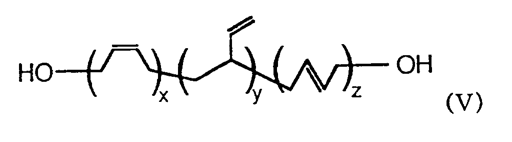
where x, y and z are numbers. These particular hydroxyl-terminated polybutadiene oligomer reactants may be prepared, in one embodiment, according to the methods of US-A-5043484 and US-A-5159123. The oligomers contain hydroxyl groups that are in predominantly primary, terminal positions on the main hydrocarbon chain and are allylic in configuration. Ordinarily, at least 1.8 hydroxyl groups are present per
molecule on the average, and advantageously there are at least from 2.1 to 3 or more hydroxyls per polymer molecule, e.g. 2.1 to 2.8. The diene polymer has most of its unsaturation in the main hydrocarbon chain, such that x plus z in formula (V) is greater than y. Structure (V) should not be understood as implying that the polymers are necessarily in blocks, but that the *cis*-1,4; *trans*-1,4 and vinyl (1,2) unsaturation is usually distributed throughout the polymer molecule. This is true for all such formulae herein. The letter x may represent a number sufficient to give a *trans*-1,4 unsaturation content of 40-70 percent; y may be a number sufficient to give a 1,2-vinylic unsaturation content to the polymer in the range of 10-35 percent, while z may be sufficient to provide a *cis*-1,4-unsaturation of 10-30 percent, in one embodiment. Often the polymer will contain largely *trans*-1,4-units, e.g. 50-65 percent and 15-25 percent *cis*-1,4-units, with 15-25 percent 1,2-units. Branching may also occur in the above polymers, especially those prepared at higher temperatures; ether and carbonyl linkages may appear in the lower molecular weight oligomer fractions.

The number average molecular weight of the HTPB oligomers of formula (V) is ordinarily in the range of 100 to 20,000, and the hydroxyl (-OH) content of said products is in the range of 0.1 to 20 meq/g, or higher. Preferably, the number average molecular weight is in the range 200-5000 and the hydroxyl content is in the range of 0.05 to 10 meq/g.

These oligomers may be hydroxyl-terminated liquid homopolymers of butadiene to which two to twenty moles of an alkylene oxide, such as ethylene oxide, propylene oxide, butylene oxide, or mixtures thereof, have been added. Alternatively, the intermediates could be hydroxyl-terminated liquid copolymers of butadiene and other vinyl monomers with similar alkylene oxide substituents. As noted previously the materials are not primary hydroxyl-terminated, so that the amination may proceed most completely. That the terminating hydroxyl is secondary is ensured by capping the precursor with an alkylene oxide other than ethylene oxide, i.e. one having at least 3 carbon atoms. With a secondary hydroxyl termination, the final degree of amination may range from 25 to 90%, or greater.

The simultaneous hydrogenation and amination is conducted over a nickel/copper/chromium/molybdenum catalyst. Most preferably, this catalyst contains nickel oxide, copper oxide, chromium oxide and molybdenum oxide, where the nickel oxide is the single greatest component of the active catalyst and the other components are promoters in lesser proportion. In one embodiment, the catalyst has the following proportions: 10 to 50 wt.% of nickel oxide, 1 to 20 wt.% of copper oxide, 0.1 to 10 wt.% of chromium oxide and 0.1 to 10 wt.% of molybdenum oxide. The catalyst is preferably supported on an inert support, such as an oxide selected from the Group IIIA, IVA or IVB of the Periodic Table. Supports may include alumina, magnesia, silica, zirconia and titania, as well as mixtures thereof. The preferred support is alumina.

Hydrogen and ammonia are used in excess as the co-reactants in the last, concurrent step. The molar ratio of hydrogen to alkoxylated liquid polymer is at least 50:1, while the molar ratio of ammonia to alkoxylated liquid polymer is at least 10:1. The alkoxylated liquid polymers are preferably reacted with hydrogen and ammonia simultaneously in the presence of an inert solvent. The solvent should be one which is stable and substantially chemically inert to the components of the reaction system at the reaction temperatures to be employed. Suitable solvents include, but are not limited to, tertiary aliphatic and aromatic alcohols, such as tertiary butanol; 2-methyl-2-butanol; 2-methyl-2-pentanol; triphenylmethanol and the like. The preferred solvent is *t*-butanol.

Unlike the method of US-A-4994621 where only part of the double bands are hydrogenated, essentially all of the double bonds are hydrogenated using the method of the present invention. Additionally, the degree of amination using the instant method is also higher than that achieved using the method of US-A-4994621. The present method gives essentially saturated, aminated, alkoxylated polybutadienes with a high degree of amination. By "essentially saturated" is meant at least 90% saturation, preferably at least 96% saturation. By high degree of amination is meant at least 80% amination.

The temperature range for the concurrent hydrogenation and amination procedure may be from 170 to 250°C and the pressure range may be from 0.79 to 34.57 MPa (100 to 5000 psi), preferably from 3.55 to 27.68 MPa (500 to 4000 psi). The simultaneous hydrogenation and amination may be conducted batchwise, or in a continuous fixed bed or slurry reactor. The products were analyzed by ¹H and ¹³C NMR, chromatography (GPC) and wet chemical techniques.

The resulting saturated polyoxyalkylene polyamines can be used as curing agents to provide epoxy resins that are more water-resistant, that it, hydrophobic, than those obtained using previously known polyamines. The invention will be illustrated further with reference to the following non-limiting Examples.

### EXAMPLE 1

Example 1 illustrates the step of synthesizing the alkoxylated hydroxyl-terminated polybutadienes using a tetramethylammonium hydroxide catalyst, where starting with a HTPB of about 1390 molecular weight, 80% of the -CH₂OH termination has been alkoxylated with propylene oxide to provide secondary hydroxyl termination.

13.62 Kg (thirty pounds) of Atochem R-20LM (a 1390 molecular weight polybutadiene polyol) and 204 g of tetramethylammonium hydroxide pentahydrate were charged into a 38 litre (ten-gallon) reactor which was then purged with prepurified nitrogen. 1.97 Kg (4.35 lbs) of propylene oxide was then reacted at 80-85°C at 0.25 MPa (22 psig) over a 0.5 hour period. The reaction mixture was then digested for two hours at 80-85°C and one hour at 125°C. It was subsequently vacuum stripped to a minimum pressure and purged with nitrogen for 30 minutes. After cooling to 50°C the product was drained from the reactor. Properties of the finished product were as follows:

| | |
|---|---|
| Total amine, meq/g | 0.019 |
| Hydroxyl no., mg KPH/g | 114 |
| Water, wt.% | 0.01 |
| pH in 10:6 isopropanol/water | 8.5 |

| Viscosity °C (°F), 10⁻⁶ m²/sec. (cs) | |
|---|---|
| 25 (77) | 1651 |
| 38 (100) | 763 |

The NMR spectra indicated that 80% of the -CH₂OH termination of the Atochem R-20LM had reacted with the propylene oxide, and the olefinic content of the final product was 45.8%.

### EXAMPLE 2

Example 2 illustrates concurrent amination and hydrogenation of the polybutadiene polyol of Example 1, using a Ni-Cu-Cr-Mo on alumina catalyst at 200°C, in the presence of an excess ammonia and hydrogen mixture where the initial polybutadiene polyol/ammonia/hydrogen molar ratio is 1:190:285. The resulting product, on analysis showed:
90% amination, by wet chemical analysis.
91% primary amine, by wet chemical analysis.
>80% saturation of the polybutadiene double bonds, by NMR.
Further, this Example illustrates the selective amination of the polybutadiene poly of Example 1.

To a 550 cm³ capacity, tubular, continuous reactor system set with temperature, pressure and feed rate controls and operated in the upflow mode, was charged a nickel oxide/chromium oxide/copper oxide/molybdenum oxide catalyst on an alumina support and having a nickel content of about 38 wt.%, 5.5.% copper, 0.95% chromium and 0.57% molybdenum. Said catalyst was in extruded form having 0.8mm (1/32") diameter. A 50:50 mixture of the polybutadiene polyol of Example 1 plus tertiary butanol solvent was then charged to the reactor system at a rate of 45.4 g/h (0.1 lb/h), along with ammonia 45.4 g/h (0.1 lb/h) and hydrogen (90 l/h) such that the molar ratio of polybutadiene polyol to ammonia to hydrogen was approximately 1:190:285. The reactor was then heated to 200°C and a back pressure of about 19.1 MPa (2750 psi) maintaining during hydrogenation/amination of said polyol.

The crude reactor effluent samples A to C were stripped at 100°C and 0.67 KPa (<5 mm Hg) for 1-2 hours. Typical product had the following properties:

| | |
|---|---|
| Total acetylatables | 1.78 meq/g |
| Total amine | 1.61 meq/g |
| Primary amine | 1.47 meq/g |
| Melting point | 100°C |
| Number average mol. wt. (by GPC) | 1370 |
| Dispersity | 2.4 |

The NMR spectra of this product indicated that the olefinic content was down to 4.2%. This indicates a product about 96% saturated (91% of available double bonds saturated). This must be contrasted with the highest aminated product described in US-A-4994621 (Example 4) where similar analyses show the residual olefinic content is 14.2% and only 29% of the available double bonds are saturated. Thus, unlike only partial unsaturation achieved according to US-A-4994621 essential saturation is achieved reliably here, defined as 90% or better saturation. Additionally, the degree of amination achieved in the US-A-4994621 (Example 4) material was on the order of only 60-62%, while here amination of at least 80% or greater is achievable. More information is given below, in Table I, for the products of Example 2, basis wet chemical, MP, GPC and NMR analyses.

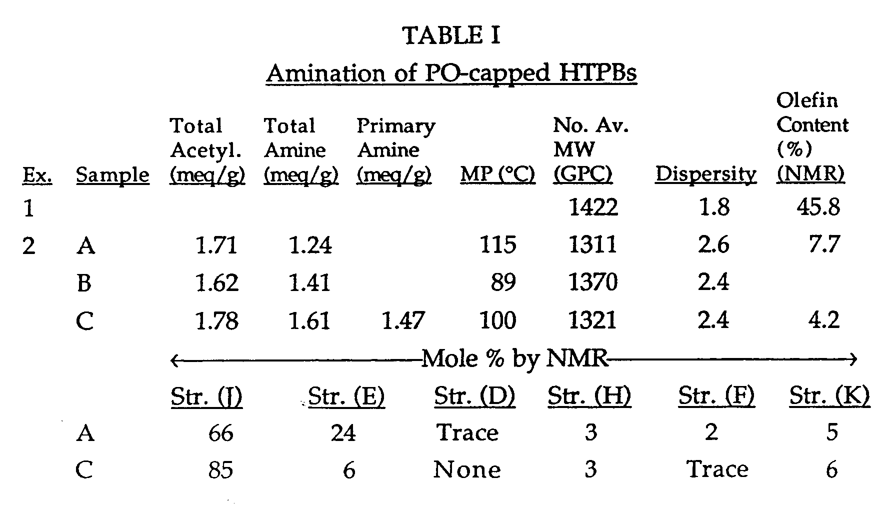

For the most part, ¹H NMR indicates that structures (A) and (B) have had PO added to them, and then both hydrogenation and the PO-terminal groups amination has taken place (see the diagram, below). The average PO chain length is evidently short (1.5 moles average) as expected. Structure (C), which with uncapped HTPBs has in the past been difficult to aminate (it is usually hydrogenated), appears in these samples to be mostly hydrogenated and aminated. This conclusion is based on the bands believed to be due to structure (K). Some (H) and (L) may have been formed, but so far it has not been possible to distinguish these from structures (E) and (J). The predominant products have structure (J). 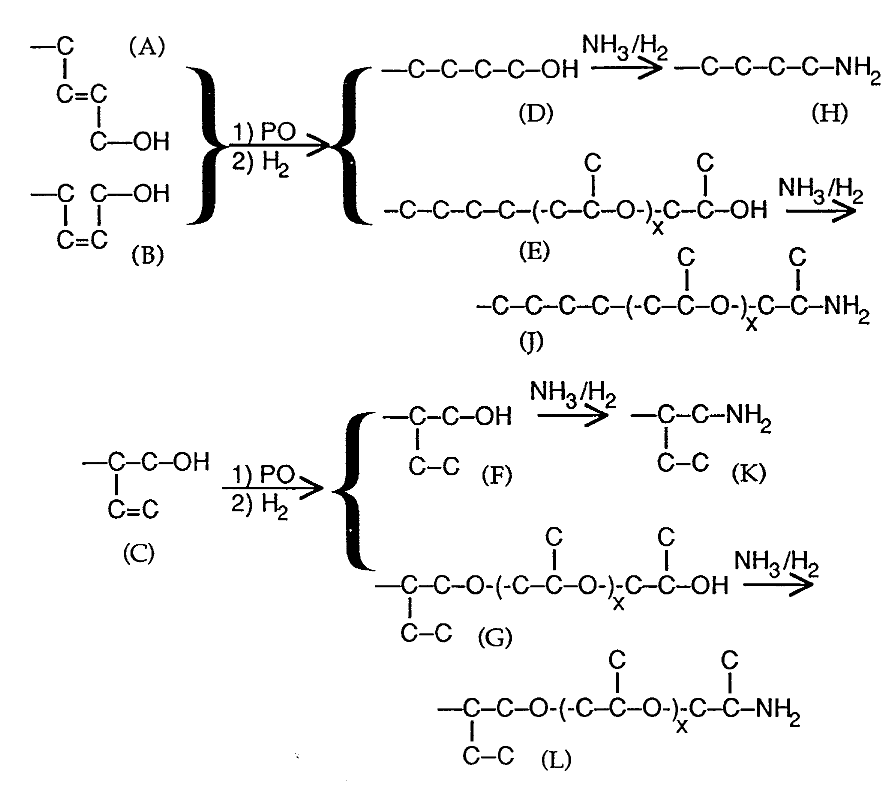
The major reaction paths during the amination portion of the concurrent hydrogenation/amination of alkylene oxide capped HTPBs are summarized above.

### EXAMPLE 3

This Example illustrates the use of the saturated polyamine products of Example 2 in the preparation of a flexible, polyurethane foam. The formulation, details of preparation and foam properties are shown below:

| Formulation | |
|---|---|
| Poly G 32-48 | 100 |
| Goldschmidt B-8231 | 1.3 |
| Water | 5.1 |
| MeCl₂ | 5.0 |
| Firemaster 642 | 9.0 |
| Dabco T-10 | 0.5 |
| Saturated polyamine from Ex. 2 | 10.0 |
| Toluene diisocyanate | 63.55 |

| Preparation | |
|---|---|
| Cream time, sec. | 10 |
| Rise time, sec. | 136 |
| Post cure, °C (h) | 100(1) |

| Property | |
|---|---|
| Density, Kg/m³ (lb/ft³) | 22.43(1.40) |

### EXAMPLES 4-14

Following the procedure of Example 2, amination of PO-capped HTPBs has been demonstrated over a wide range of experimental conditions using the same 550 cm³ capacity unit and the Ni-Cu-Cr-Mo on alumina catalyst. A summary of the results, particularly the wet chemical, GPC and ¹H NMR data for each individual product, is set out in the accompanying Tables II and III. The operating parameters varied during this study included:
a) Polyol feed rate [1:1 with t-butanol, total rates 23-227 g/h (0.05-0.5 lb/h)]
b) Ammonia feed rate [9-22.7 g/h (0.02-0.5 lb/h)]
c) Hydrogen feed rate (5-300 1/h)
d) Operating temperature (185-215°C)

All aminated products, after work-up, were solids - indicating extensive hydrogenation of the HTPB olefinic backbone. The highest functionality (1.90 meq/g acetylatables) was achieved at moderate hydrogen feed rates (30l/h, Example 11). In this case, the amination level was 82% and the primary amine content 94%. Example 10 was carried out at a lower polyol feed rate. However, the low total acetylatables value for this sample (1.67 meq/g) indicates some loss of end-group functionality under these conditions. Not surprisingly, the highest level of unsaturation (18.4%) was realized at the lowest hydrogen feed rate (Example 12). However in this case, the product amine level was only 1.10 meq/g, although the estimated primary amine value was 96%.

Degradation of the Ni-Cu-Cr-Mo catalyst, indicated by gray fines in the product effluent, appeared to be a problem only at the highest feed rates (e.g. in Example 7). Surprisingly, total amine and primary amine values (1.22 and 1.13 for Ex. 7) were depressed under those conditions.

### EXAMPLE 15

Following the procedures of Example 2, a twenty-mole PO adduct of Atochem R-20LM polyol, having a hydroxyl number of 64 mg KOH/g was aminated under standard conditions with a fresh charge of the Ni-Cu-Cr-Mo on alumina 0.8 mm (1/32") extruded catalyst. Product analyses for Samples A to L, summarized in Tables IV and V, show typically about 82% amination levels with 96% primary amine content and 3.4% residual olefin (e.g. Sample D). The ¹H NMR data also indicate 85-89 mole percent secondary alkyl primary amine termination for these crude, stripped, products (see Table V). While the amination level in Tables IV and V is a little lower than that reported for the 5 molar PO adducts in Table I, the product series in Tables IV and V is noteworthy in that their NMR spectra do not exhibit measurable primary alkyl, primary amine functionality that would be indicative of uncapped hydroxyl end groups in the Example 15 feedstock (see the diagram of the major reaction paths, above).

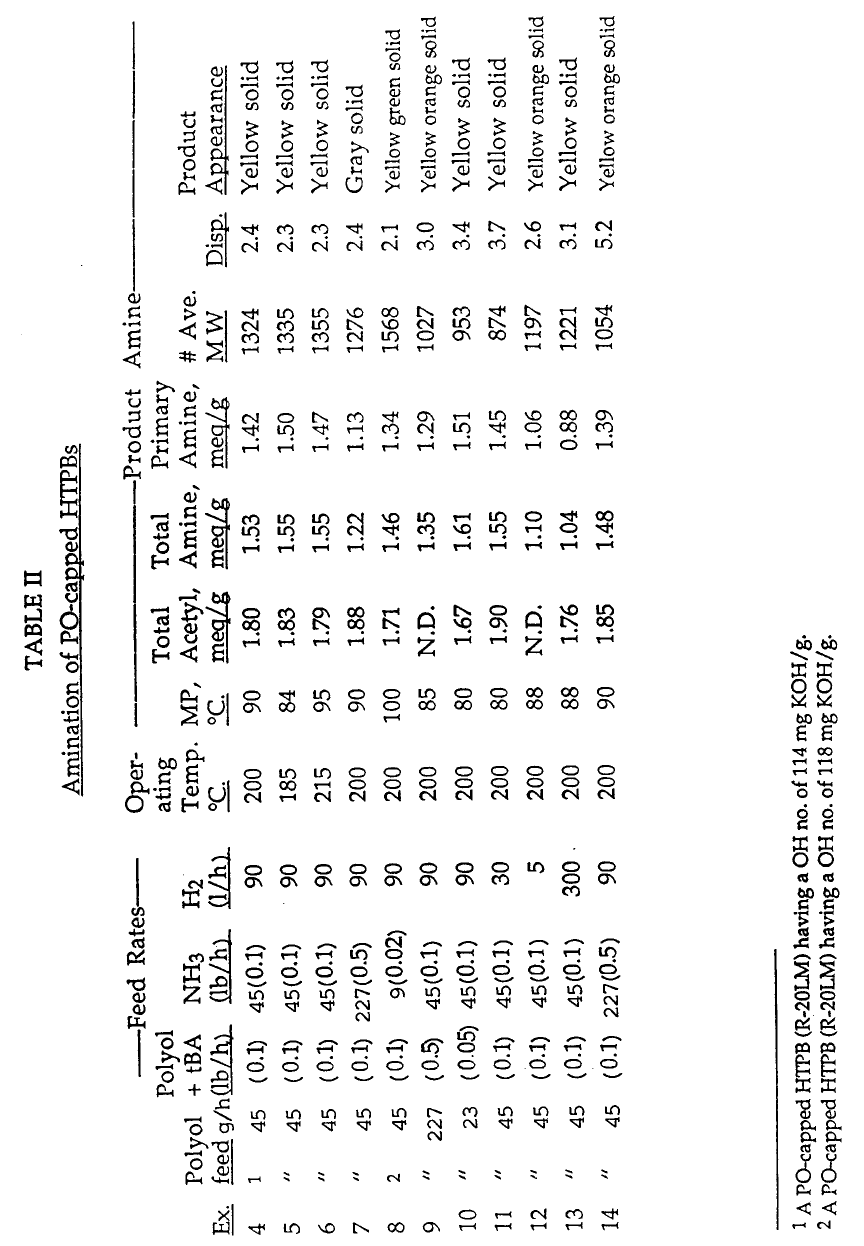

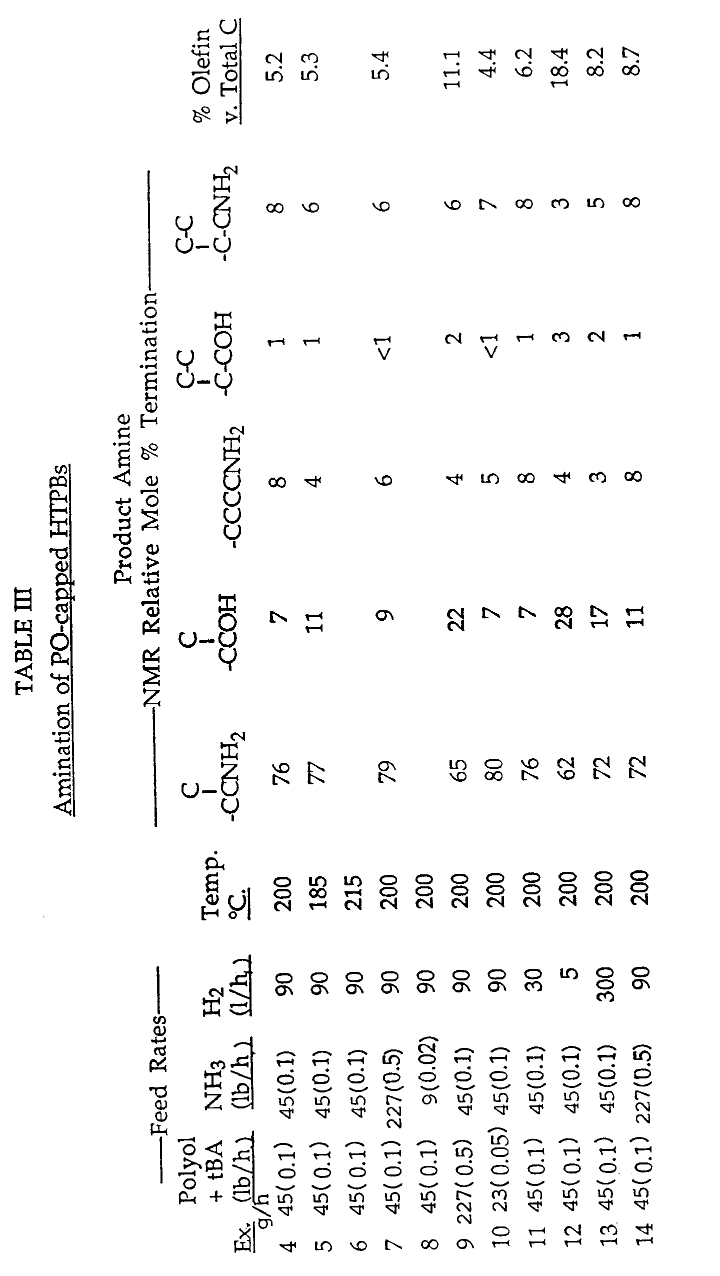

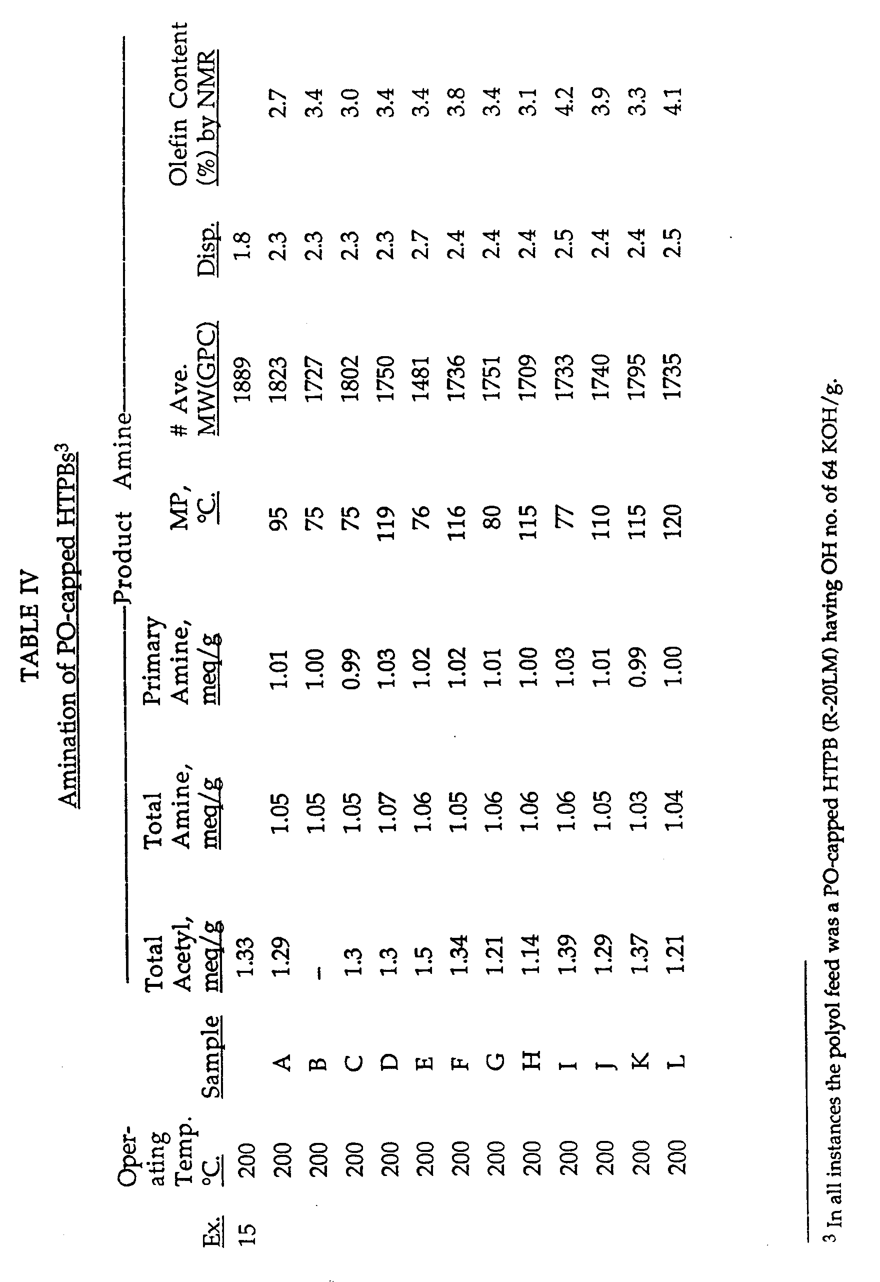

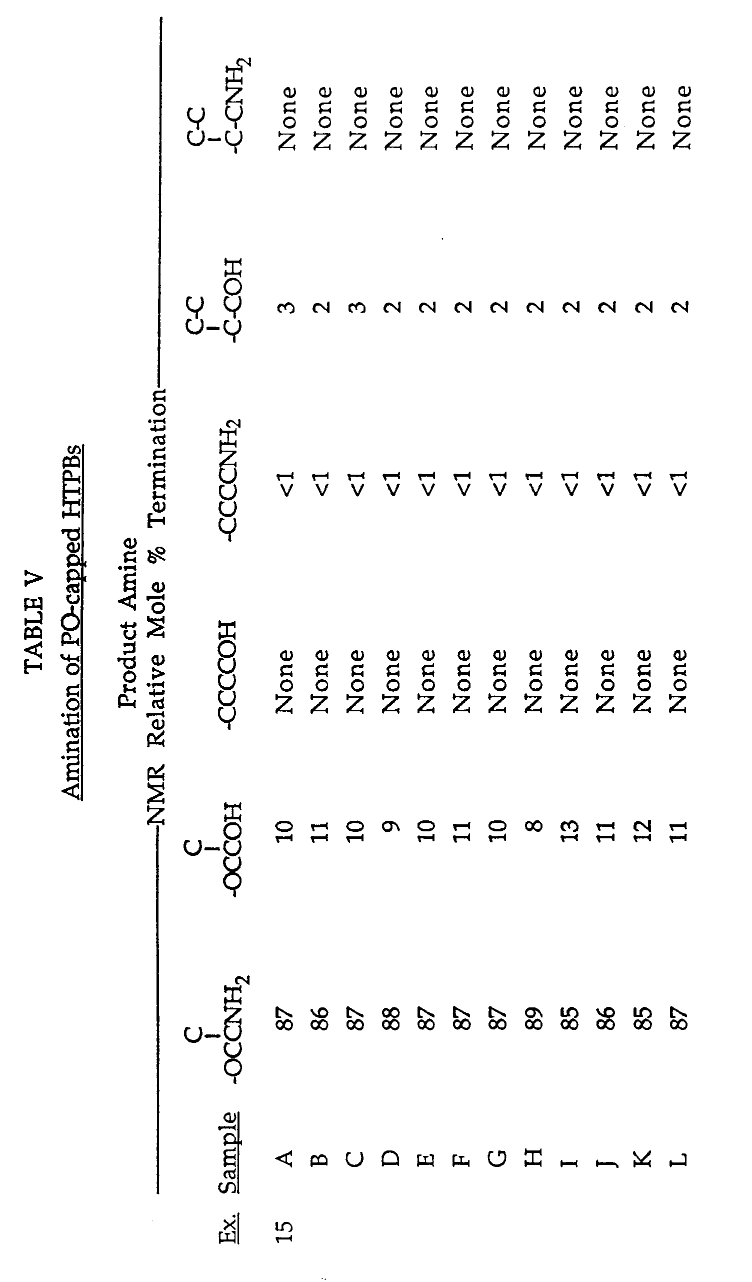

### EXAMPLES 16-21

In a manner similar to Example 2 a primary hydroxyl-terminated polybutadiene (HTPB), R45HT product from Atochem, was continuously and simultaneously aminated and hydrogenated using the Ni-Cu-Cr-Mo catalyst of Example 2 and a Ni-Cu-Cr catalyst for comparison within the definition of US-A-4994621 at the indicated temperatures. The HTPB had not been reacted with an alkylene oxide. The products had the following properties:

**TABLE VI**

| Simultaneous Amination/Hydrogenation of HTPB | | | | | | | | |
|---|---|---|---|---|---|---|---|---|
| Ex. | Catalyst | Operating Temp. (°C.) | Total amine (meq/g) | Primary amine (meq/g) | No. Av. MW (gpc) | NMR ¹H (%) | | |
| | | | | | | Total olefin | Total amine | |
| HTPB unreacted | | | | | 2132 | 34.9 | | |
| 16 | Ni-Cu-Cr-Mo | 190 | 0.44 | 0.34 | 2243 | 16.3 | 0.72 | |
| 17 | " | 200 | 0.45 | 0.31 | 2679 | 16.1 | 0.75 | |
| 18 | " | 210 | 0.48 | 0.34 | 2752 | 14.5 | 0.75 | |
| 19* | Ni-Cu-Cr | 190 | 0.28 | 0.14 | 3308 | 18.0 | 0.60 | |
| 20* | " | 200 | 0.41 | 0.28 | 3067 | 16.7 | 0.67 | |
| 21* | " | 210 | 0.41 | 0.28 | 3170 | 16.1 | 0.66 | |

| | | | | | | | |
|---|---|---|---|---|---|---|---|
| * For Comparison | | | | | | | |

The results in Table VI show that the Ni-Cu-Cr-Mo catalyst of this invention gives the following results as compared with the prior art Ni-Cu-Cr catalyst over the same temperature range (190-210°C):
1) The inventive process gives more highly aminated products;
   compare total amine by wet chemical techniques (column 4) for Examples 16-18 (0.44; 0.45; 0.48) with Comparative Examples 19-21 (0.28; 0.41; 0.41). See also total amine by ¹H NMR technique (col. 8) where Examples 16-18 of this invention gave values of 0.72; 0.75 and 0.75, respectively, compared with values of 0.60; 0.67 and 0.66, respectively.
2) The invention process gives greater primary amine product;
   compare Examples 16-18 with Comparative Examples 19-21 for column 5 of Table VI (values of 0.34; 0.31 and 0.34, respectively v. 0.14; 0.28 and 0.28 respectively).
3) The inventive process gives less competing HTPB oligomerization, i.e. less of an undesirable increase in molecular weight during amination. This will be seen from column 6 in Table VI, where the molecular weights for Examples 16-18 were 2243; 2679 and 2752, respectively, as compared with 3308; 3067 and 3170, respectively, for Comparative Examples 19-21.
4) The inventive process also gives greater reduction in the percentage of total olefin found, which is understood to mean greater hydrogenation. Compare the values in column 7 of Examples 16-19 of 16:3; 16.1 and 1.45, respectively, which are lower (greater hydrogenation) than those of 18.0; 16.7 and 16.1 of Examples 19-21, respectively.

### EXAMPLES 22-23

In a manner similar to Example 2, a primary hydroxyl-terminated polybutadiene (HTPB), R45HT product from Atochem, that had been capped with 4.0 moles of propylene oxide in a manner similar to Example 1, was continuously and simultaneously aminated and hydrogenated using the Ni-Cu-Cr-Mo catalyst of Example 2 and a Ni-Cu-Cr catalyst at the indicated temperatures. The catalysts were the same as for Examples 16-21. The operating conditions for Examples 22-23 were as follows:

| | | |
|---|---|---|
| Temperature | | 185°C |
| Pressure | | 2000 psi |
| Catalyst charge | | 500 cm³ |
| Feed Rates | PO-capped HTPB/t-butanol (1:1 weight ratio) | 163.4g/h / 0.36lb/h |
| | NH₃ | 163.4g/h / 0.36lb/h |
| | H₂ | 171/h |

The products had the following properties:

**TABLE VII**

| Simultaneous Amination/Hydrogenation of PO-Capped HTPB | | | | | | |
|---|---|---|---|---|---|---|
| Ex. | Catalyst | Total acetyl. (meq/g) | Total amine (meq/g) | Primary amine (meq/g) | No. Av. MW (gpc) | Dispersity |
| | PO-capped HTPB unreacted | <1.94 | | | 2765 | 1.9 |
| 22 * | Ni-Cu-Cr | | 0.36 | 0.32 | 3394 | 2.0 |
| 23 | Ni-Cu-Cr-Mo | | 0.90 | 0.90 | 2363 | 2.7 |

| | | | | | | |
|---|---|---|---|---|---|---|
| * For Comparison | | | | | | |

Table VII demonstrates that in Example 23, the Ni-Cu-Cr-Mo catalyst of this invention gives the following results as compared with the Ni-Cu-Cr catalyst for propylene oxide-capped HTPBs used in Example 22:
1) The inventive claimed process gives more highly aminated products;
   compare total amine by wet chemical techniques (column 4) for Example 23 (0.90) using the catalyst of this invention with Comparative Example 22 (0.36). This increase is nearly threefold.
2) The invention claimed process gives greater primary amine product;
   compare Example 23 (0.90) with Comparative Example 22 for column 5 of Table VII (0.32). Again, this increase is nearly threefold.
3) The inventive claimed process gives less competing HTPB oligomerization, i.e. less of an undesirable increase in molecular weight during amination. Please refer to column 6 in Table VII which indicates that the molecular weight of the Ex. 22 product is 3394, considerably more than the molecular weight of 2393 for Example 23 (an increase of about 44%).

Many modifications may be made in the process of this invention as defined in the appended claims. For example, one skilled in the art may discover that particular compounds or proportions thereof, which may not be explicitly recited herein, but which are nevertheless anticipated, would give desirable results. A certain combination of reaction temperatures, reactant proportions, etc. may be found to have particular advantages.

### GLOSSARY

- Dabco T-10: 50% stannous octoate in dioctyl phthalate; tin catalyst made by M&T Chemicals.
- Firemaster 642: A halogenated phosphate fire retardant made by Great Lakes Chemicals.
- Goldschmidt B-8231: A silicone oil surfactant made by Goldschmidt.
- Poly G 32-48: A 3500 molecular weight glycerol-derived triol having about 10% internal ethylene oxide made by Olin Chemicals.
- tBA: Tertiary butanol.

## Claims

1. A process for making saturated, aminated alkoxylated polymers by reductive amination of an alkyoxylated liquid polymer obtained by alkoxylating a hydroxyl-terminated polybutadiene (HTPB) with one or more alkylene oxides having at least three carbon atoms to provide secondary terminal hydroxyl groups, characterised in that the alkoxylated liquid polymer is contacted with hydrogen and ammonia in the presence of a catalyst whose active components are nickel oxide, copper oxide, chromium oxide and molybdenum oxide, and in that the molar ratio of hydrogen to ammonia to secondary hydroxyl groups on the alkoxylated liquid polymer is at least 50 : at least 10:1.

2. A process according to Claim 1 characterised in that the HTPB is alkoxylated with propylene oxide, butylene oxide or a mixture thereof.

3. A process according to Claim 1 or 2 characterised in that the catalyst comprises 10 to 50 wt.% of nickel oxide, 1 to 20 wt.% of copper oxide, 0.1 to 10 wt.% of chromium oxide and 0.1 to 10 wt.% of molybdenum oxide.

4. A process according to any one of Claims 1 to 3 characterised in that catalyst is supported on alumina, silica, zirconia, magnesia, titania or a mixture thereof.

5. A process according to any one of Claims 1 to 4 characterised in that reductive amination is conducted at a temperature of 170 to 250°C.

6. A process according to any one of Claims 1 to 5 characterised in that reductive amination is conducted at a pressure of 0.79 to 34.57 MPa (100 to 5000 psi).

7. A process according to any one of Claims 1 to 6 characterised in that reductive amination is conducted in the presence of *t*-butanol as an inert solvent.

8. Saturated, aminated alkoxylated polymers of the formula: 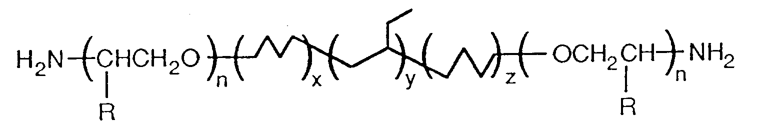 wherein each R is independently alkyl of 1 to 4 carbon atoms;
each n is independently from 1 to 100; and
x, y and z are numbers,
the number average molecular weight of the hydrocarbon backbone being from 200 to 5000.
